(19) Europäisches Patentamt
European Patent Office
Office européen des brevets

(11) Veröffentlichungsnummer: **0 041 172**
**B1**

# (12) EUROPÄISCHE PATENTSCHRIFT

(45) Veröffentlichungstag der Patentschrift: **18.05.83**

(51) Int. Cl.³: **C 07 C 103/44**

(21) Anmeldenummer: **81103790.2**

(22) Anmeldetag: **18.05.81**

## (54) Verfahren zur Herstellung von Pivaloylessigsäure-2-chlor-5-(4-(2,4-di-tert.-amyl)-phenoxybutyramido)-anilid.

(30) Priorität: **29.05.80 DE 3020445**
**29.05.80 DE 3020442**

(43) Veröffentlichungstag der Anmeldung:
**09.12.81 Patentblatt 81/49**

(45) Bekanntmachung des Hinweises auf die Patenterteilung:
**18.05.83 Patentblatt 83/20**

(84) Benannte Vertragsstaaten:
**DE FR GB**

(56) Entgegenhaltungen:
**keine**

(73) Patentinhaber: **BAYER AG, Zentralbereich Patente, Marken und Lizenzen, D-5090 Leverkusen 1, Bayerwerk (DE)**

(72) Erfinder: **Blank, Heinz Ulrich, Dr., Am Geusfelde 35, D-5068 Odenthal (DE)**
Erfinder: **Werner, Friedrich, Dr., Petersenstrasse 1, D-5000 Köln 91 (DE)**
Erfinder: **Wolters, Erich, Dr., Streffenweg 22, D-5162 Niederzier (DE)**
Erfinder: **Müller, Karl Walter, Dr., Bertha von Suttnerstrasse 29, D-5090 Leverkusen 1 (DE)**
Erfinder: **Rottloff, Günter, Semmelweiss-Strasse 70, D-5000 Köln 80 (DE)**

**EP 0 041 172 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Pivaloylessigsäure-2-chlor-5-[4-(2,4-di-tert.-amyl)-phenoxybutyramido]-anilid

Die Erfindung betrifft ein Verfahren zur Herstellung von Pivaloylessigsäure-2-chlor-5-[4-(2,4-di-tert.-amyl)-phenoxybutyramido]-anilid.

Pivaloylessigsäure-2-chlor-5-[4-(2,4-di-tert.-amyl)-phenoxybutyramido]-anilid ist bekannt und dient als Zwischenprodukt für die Herstellung von Farbfilmkomponenten (US 3 265 506, US 3 384 657, DE-AS 11 24 356). Die Synthese für dieses wichtige Zwischenprodukt besteht darin, dass man 4-(2,4-Di-tert.-amyl)-phenoxybuttersäurechlorid mit 4-Chlor-3-nitroanilin in Essigsäure umsetzt. Danach wird das erhaltene 4-(2,4-Di-tert.-amyl)-phenoxy-buttersäure-4-chlor-3-nitroanilid in Ethanol mit Wasserstoff in Gegenwart von Raney-Nickel zum 4-(2,4-Di-tert.-amyl)-phenoxybuttersäure-3-amino-4-chloranilid reduziert und anschliessend mit Pivaloylessigsäureethylester zum Pivaloylessigsäure-2-chlor-5-[4-(2,4-di-tert.-amyl)-phenoxybutyramido]-anilid umgesetzt.

Das dargestellte Verfahren hat gravierende Nachteile. So wird der erste Syntheseschritt in einem grossen Überschuss an Essigsäure durchgeführt und die Isolierung des 4-(2,4-Di-tert.-amyl)-phenoxybuttersäure-4-chlor-3-nitroanilid gelingt in günstiger Weise nur durch Einbringen der Reaktionslösung in die etwa dreifache Menge Wasser. Nach der Filtration des Produktes verbleibt eine mit grosser Kohlenstoff-Belastung behaftete Mutterlauge, die aus ökologischer Sicht eine starke Kostenbelastung dieses Verfahrens ist. Weiterhin kann das nach der Filtration erhaltene Produkt erst durch zweifaches Umlösen in eine für die Weiterverwendung ausreichende Form gebracht werden. Auch die anschliessende Reduktion ergibt ein Rohprodukt, das erst durch zweifaches Umlösen aus Cyclohexan in genügender Reinheit anfällt. Obwohl die Zwischenprodukte mehrfach gereinigt werden, gelingt es nicht, das Pivaloylessigsäure-2-chlor-5-[4-(2,4-di-tert.-amyl)-phenoxybutyramido]-anilid in der nachfolgenden Reaktion durch Umsetzen des 4-(2,4-Di-tert.-amyl)-phenoxybuttersäure-3-amino-4-chloranilids mit Pivaloylessigsäure-ethylester in reiner Form zu isolieren. Auch hier sind Umlösungen erforderlich.

Für die spätere Verwendung dieses Produktes als Farbfilmkomponente ist jedoch klar ersichtlich eine hohe Reinheit erforderlich. Weiterhin ist es dem Fachmann bekannt, dass das Umlösen einer Substanz nicht nur arbeitsaufwendig ist, sondern auch zu grossen Substanzverlusten führt.

Es bestand demnach die Notwendigkeit, die Synthese des Pivaloylessigsäure-2-chlor-5-[4-(2,4-di-tert.-amyl)-phenoxybutyramido]-anilids einfacher und in ökologisch vorteilhafter Weise zu gestalten.

Es wurde nun ein Verfahren zur Herstellung von Pivaloylessigsäure-2-chlor-5-[4-(2,4-di-tert.-amyl)-phenoxybutyr-amido]-anilid gefunden, das dadurch gekennzeichnet ist, dass man Pivaloylessigsäure-2-chlor-5-nitro-anilid mit Wasserstoff katalytisch in Gegenwart eines Amins oder Phosphins in einem inerten Lösungmittel bei einer Temperatur von 0 bis 150°C umsetzt und die dabei erhältliche korrespondierende Aminoverbindung danach weiter mit einem Halogenid der 4-(2,4-Di-tert.-amyl)-phenoxybuttersäure bei einer Temperatur von -50 bis +150°C in einem organischen Lösungsmittel, gegebenenfalls in Gegenwart einer Base, umsetzt.

Das Pivaloylessigsäure-2-chlor-5-nitro-anilid kann beispielsweise nach einem bisher nicht veröffentlichten Verfahren durch Kondensation von Pivaloylessigsäuremethylester und 2-Chlor-5-nitro-anilin hergestellt werden.

Pivaloylessigsäuremethylester ist bekannt und kann beispielsweise durch Erhitzen von Pivaloylbrenztraubensäuremethylester auf etwa 175° unter Decarbonylierung hergestellt werden (US 2 527 306). 2-Chlor-5-nitro-anilin ist bekannt (Ber. Deut. Chem. Ges. 33, 3057 [1900], bes. S. 3062) und kann durch Nitrieren von o-Chloranilin in der 10fachen Gewichtsmenge Schwefelsäure bei 0°C mit der berechneten Menge Salpetersäure vom spez. Gew. 1,5 in überschüssiger Schwefelsäure erhalten werden.

Das als Zwischenprodukt im erfindungsgemässen Verfahren auftretende Pivaloylessigsäure-2-chlor-5-amino-anilid ist aus DE-OS 25 15 771 bekannt, ohne dass dort ein Verfahren zu seiner Herstellung beschrieben ist.

Die genannten Reaktionsschritte können beispielsweise durch die folgenden Reaktionsgleichungen dargestellt werden:

1.

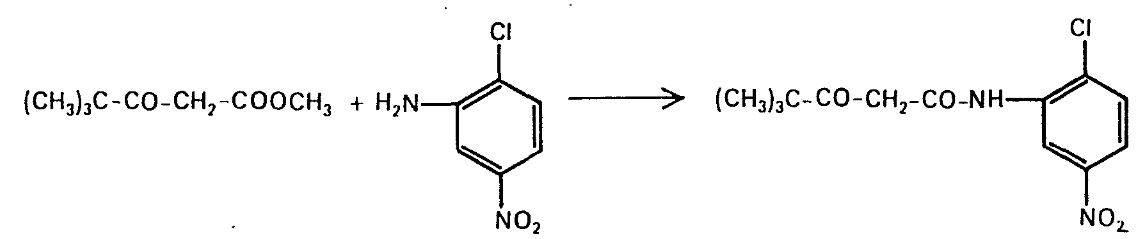

2.

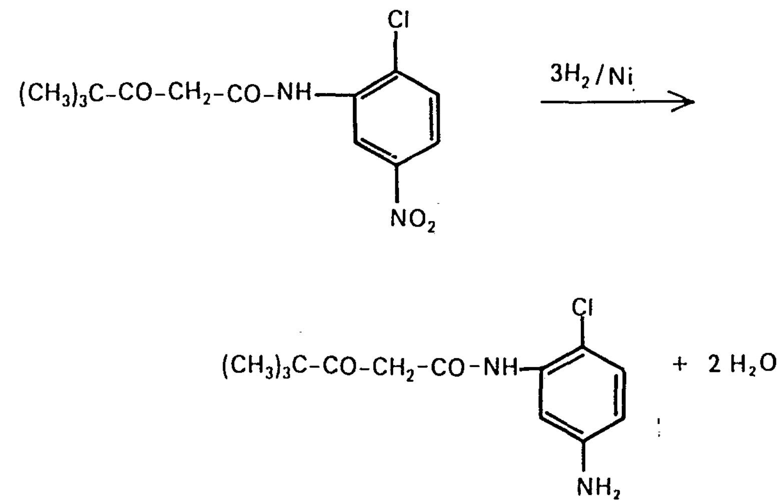


3.

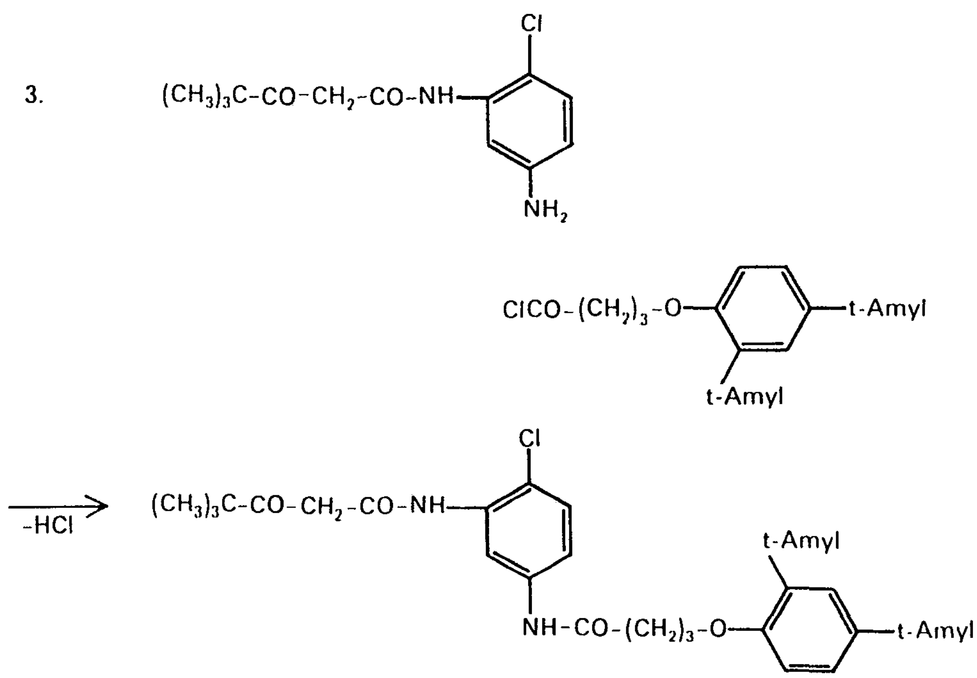


Das 4-(2,4-Di-tert.-amyl)-phenoxy-buttersäure-chlorid ist ebenfalls bekannt (US 3 265 506) und kann nach Verfahren des Standes der Technik aus γ-Butyrolacton und 2,4-Di-tert.-amyl-phenol und anschliessender Überführung in das Säurechlorid hergestellt werden. Als Halogenid der 4-(2,4-Di-tert.-amyl)-phenoxybuttersäure kann jedoch auch das Säurebromid eingesetzt werden. Bevorzugt wird das Säurechlorid eingesetzt.

Die Umsetzung mit Wasserstoff wird an einem an sich bekannten Hydrierkatalysator durchgeführt. Als Hydrierkatalysator können erfindungsgemäss alle dem Fachmann hierfür bekannten Katalysatoren verwendet werden, wie beispielsweise Edelmetallkatalysatoren, wie Platin oder Palladium, oxydische Katalysatoren, wie Kupferchromit oder Kobalt oder Nickel enthaltende Katalysatoren. Diese Katalysatoren können sowohl einzeln als auch im Gemisch eingesetzt werden, sie können als Trägerkatalysatoren oder ohne einen Träger verwendet werden. Bevorzugt ist erfindungsgemäss die Verwendung eines Kobalt oder Nickel enthaltenden Katalysators. Besonders bevorzugt sind die Kobalt oder Nickel enthaltenden Katalysatoren in der Form von Raney-Katalysatoren, wie Raney-Nickel, Raney-Kobalt, Raney-Eisen-Nickel, Raney-Eisen-Kobalt-Nickel, Raney-Eisen-Kobalt. Unter diesen Raney-Katalysatoren sei in ganz besonders bevorzugter Weise Raney Nickel genannt. Der Hydrierkatalysator wird im allgemeinen in einer Menge von 1 bis 30 Gew.-%, bevorzugt 2 bis 20, besonders bevorzugt 3 bis 15 Gew.-%, bezogen auf das Pivaloylessigsäure-2-chlor-5-nitro-anilid, eingesetzt.

Der Wasserstoffdruck im erfindungsgemässen Verfahren kann in einem sehr breiten Bereich variieren, beispielsweise von 0,1 bis 300 bar. Bevorzugt wird im Bereich von 1 bis 50, besonders bevorzugt von 2 bis 30 bar gearbeitet.

Als inertes Lösungsmittel für die Umsetzung

mit Wasserstoff im erfindungsgemässen Verfahren kommen alle unter den Reaktionsbedingungen inerten Lösungsmittel in Betracht. Beispielsweise seien hiefür Kohlenwasserstoffe, wie Benzinfraktionen, Cyclohexan, Benzol, Toluol oder Xylol, Alkohole, wie Methanol, Ethanol, Propanol oder Butanol, Säuren, wie Essigsäure, Propionsäure oder Buttersäure oder Ether, wie Diethylether, Tetrahydrofuran oder Dioxan genannt. Bevorzugt ist die Verwendung eines Alkohols oder eines Kohlenwasserstoffs, besonders bevorzugt die Verwendung von Methanol oder Toluol. Es können auch Gemische der genannten inerten Lösungsmittel angewendet werden. Die erfindungsgemässe Umsetzung mit Wasserstoff wird bei einer Temperatur von 0 bis 150°C, bevorzugt von 20 bis 80°C, durchgeführt.

Die erfindungsgemässe Umsetzung mit Wasserstoff wird in Gegenwart eines Amins oder Phosphins durchgeführt. Als Amine seien beispielsweise aliphatische Amine wie Ethylamin, Diethylamin, Triethylamin, Propylamin, Diopropylamin, Tripropylamin, Butylamin, Dimethylpropylamin, Cyclohexylamin, Methyl-cyclohexyl-amin, Dimethyl-cyclohexyl-amin, aromatische Amine, wie Anilin, Diphenylamin, Triphenylamin, N-Methyl-anilin, N,N-Dimethyl-anilin und heterocyclische Amine wie Pyridin, methyl-substituiertes Pyridin, Morpholin oder Thiomorpholin, genannt. Als Phosphine seien beispielsweise aliphatische Phosphine, wie Ethylphosphin, Diethylphosphin, Triethylphosphin, Propylphosphin, Dipropylphosphin, Tripropylphosphin oder Tributylphosphin und aromatische Phosphine, wie Phenylphosphin, Diphenylphosphin oder Triphenylphosphin, genannt.

Die genannten Stickstoff- oder Phosphor-Verbindungen werden im allgemeinen in einer Menge von 0,5 bis 30, bevorzugt 1 bis 20 Gew.-%, bezogen auf eingesetztes Pivaloylessigsäure-2-chlor-5-nitro-anilid eingesetzt.

Zur Durchführung der erfindungsgemässen Umsetzung mit Wasserstoff wird das Pivaloylessigsäure-2-chlor-5-nitro-anilid gemeinsam mit dem Lösungsmittel, dem Amin oder Phosphin und dem Katalysator in einen Hydrierautoklaven gegeben. Sodann wird der gewünschte Wasserstoffdruck und die gewünschte Reaktionstemperatur eingestellt und die Hydrierung bis zur Druckkonstanz fortgeführt. Zur Aufarbeitung wird vom Hydrierkontakt abfiltriert und das Produkt durch Kristallisation aus dem eingeengten Filtrat gewonnen. Sofern das eingesetzte Amin oder Phosphin ausreichend flüchtig ist, kann das Produkt auch durch Eindampfen der filtrierten Reaktionslösung bis zur Trockne und gegebenenfalls folgender Umkristallisation gewonnen werden.

Wird die Reduktion nicht in Gegenwart eines Amins oder Phosphins durchgeführt, so kommt es zu einer Vielzahl von Nebenreaktionen. Die stärkste dieser Nebenreaktionen ist die Bildung von 2,4-Diamino-chlorbenzol unter gleichzeitiger Bildung von 2,4-Dipivaloyl-acetamido-chlorbenzol, was durch die folgende Reaktionsgleichung erläutert werden kann:

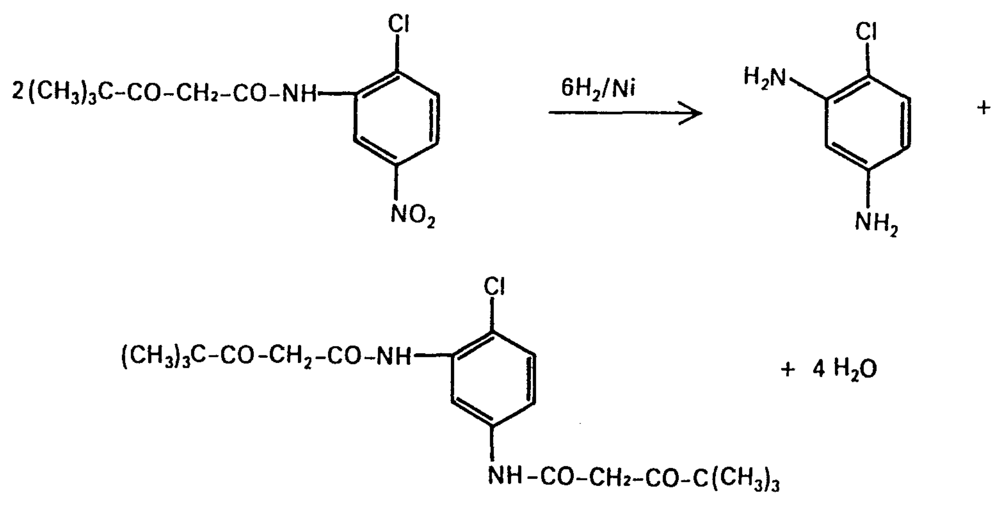


Die Unterdrückung solcher Nebenreaktionen durch den erfindungsgemässen Zusatz eines Amins oder Phosphins bewirkt demnach eine stärkere Selektivität der gewünschten Reaktion. Dies bedeutet gleichzeitig eine Erhöhung der Ausbeute auf fast quantitative Werte. Durch die Unterdrückung von Nebenprodukten wird gleichzeitig eine hohe Reinheit des gewünschten Produktes erzielt.

Die bei der Umsetzung mit Wasserstoff erhältliche Aminoverbindung wird erfindungsgemäss weiter mit einem der bereits genannten Halogenide der 4-(2,4-Di-tert.-amyl)-phenoxybuttersäure umgesetzt.

Die Aminoverbindung fällt infolge der Anwendung der erfindungsgemässen Massnahmen so rein an, dass sie ohne weitere Behandlung erfindungsgemäss weiter umgesetzt werden kann. Selbstverständlich kann jedoch diese Aminoverbindung vor der weiteren Umsetzung einer Reinigung, beispielsweise durch Umkristallisation oder Chromatographie, unterzogen werden.

Zur Vervollständigung der weiteren Umsetzung im erfindungsgemässen Verfahren sind die

Aminoverbindung und das Säurehalogenid in äquimolaren Mengen erforderlich. Es ist jedoch vorteilhaft, das Säurehalogenid in einem geringen Überschuss einzusetzen. Hierzu sei beispielsweise ein Überschuss von 1 bis 20 Mol-% über die stöchiometrische Menge hinaus genannt. Die Reihenfolge des Zusammengebens beider Stoffe kann in der erfindungsgemässen weiteren Umsetzung beliebig gewählt werden. So kann beispielsweise das Säurehalogenid vorgelegt werden und das Anilid anschliessend in das Reaktionsgemisch eingetragen werden. Es ist jedoch auch die umgekehrte Reihenfolge möglich. Schliesslich können beide Stoffe simultan dosiert werden.

Als organisches Lösungsmittel für die weitere Umsetzung seien beispielsweise Kohlenwasserstoffe, wie Benzinfraktionen, Cyclohexan, Heptan, Octan, Benzol, Toluol oder Xylol, halogenierte Kohlenwasserstoffe, wie chlorierte oder bromierte Benzinfraktionen, Chlorbenzol, Brombenzol, Dichlorbenzol oder Dibrombenzol, Ether, wie Diethylether, Diisopropylether, Tetrahydrofuran, Dioxan oder Anisol, Alkohole, wie Methanol, Ethanol, Propanol oder Butanol, oder Ester wie Essigsäureethylester, genannt. Bevorzugte Lösungsmittel sind Benzol, Toluol, Methanol, Ethanol oder Essigsäureethylester. Besonders bevorzugt ist die Verwendung von Toluol oder Methanol als Lösungsmittel. Die genannten Lösungsmittel können auch als Gemische eingesetzt werden.

Viele der genannten organischen Lösungsmittel sind identisch mit den für die erfindungsgemässe Umsetzung mit Wasserstoff genannten inerten Lösungsmitteln. Für den Fall, dass für die Umsetzung des Ausgangsstoffes mit Wasserstoff als erfindungsgemässer Zusatz eines Amins oder Phosphins ein tertiäres Amin, besonders bevorzugt Pyridin, gewählt wird und ein solches identisches Lösungsmittel eingesetzt wird, kann daher die Lösung der ersten Verfahrensstufe ohne Zwischenisolierung der Aminoverbindung zur weiteren Umsetzung mit dem Säurehalogenid verwendet werden. Zweckmässigerweise wird bei dieser bevorzugten Variante lediglich der Hydrierkontakt vor der weiteren Umsetzung abgetrennt.

Die Temperatur für die Durchführung der erfindungsgemässen weiteren Umsetzung ist innerhalb eines Temperaturbereiches von −50 bis +150°C, bevorzugt von −25 bis +110°C, wählbar.

Bei der erfindungsgemässen weiteren Umsetzung entsteht neben dem gewünschten Reaktionsprodukt zusätzlich Halogenwasserstoff, beispielsweise Chlorwasserstoff oder Bromwasserstoff. Dieser Halogenwasserstoff kann beispielsweise bei erhöhter Temperatur aus dem Reaktionsgemisch ausdestilliert werden. Er kann jedoch auch mit Hilfe eines Trägergases, beispielsweise Stickstoff, Kohlendioxid oder Argon, aus dem Reaktionsgemisch ausgeblasen werden. Weiterhin kann der entstehende Halogenwasserstoff auch durch Zugabe einer basisch reagierenden Verbindung zum Reaktionsgemisch als Salz gebunden werden. Diese letztere Variante bringt einige Vorteile, beispielsweise die Erniedrigung der Reaktionstemperatur, und ist daher vorzugsweise anwendbar. Die Anwesenheit einer basisch reagierenden Substanz ist jedoch weder für die Güte des Reaktionsproduktes noch für eine hohe Ausbeute erforderlich.

Als basisch reagierende Substanz seien beispielsweise basisch reagierende Verbindungen von Alkali- oder Erdalkalimetallen oder tertiäre organische Amine genannt. Alkalisch reagierende Verbindungen von Alkali- oder Erdalkalimetallen sind beispielsweise Lithiumhydroxid, Lithiumcarbonat, Natriumhydroxid, Natriumcarbonat, Kaliumhydroxid, Kaliumcarbonat, Calciumhydroxid, Calciumcarbonat, Strontiumhydroxid, Strontiumcarbonat, Bariumhydroxid oder Bariumcarbonat. Tertiäre organische Amine sind beispielsweise Triethylamin, N,N-Diethylanilin, N,N-Dimethylanilin, Dimethylbenzylamin, Morpholin oder Pyridin. In bevorzugter Weise wird in Gegenwart eines tertiären organischen Amins gearbeitet. Besonders bevorzugt ist der Einsatz von Pyridin.

Zur Durchführung der weiteren Umsetzung des erfindungsgemässen Verfahrens werden die Aminoverbindung und das Säurehalogenid gemeinsam mit dem Lösungsmittel zusammengegeben und die gewählte Reaktionstemperatur eingestellt. Die Reihenfolge des Zusammengebens der Ausgangsstoffe ist für den Erfolg des erfindungsgemässen Verfahrens nicht kritisch. Für den Fall, dass in Gegenwart einer basisch reagierenden Verbindung gearbeitet wird, ist es jedoch vorteilhaft, die Base mit der Aminoverbindung und dem Lösungsmittel vorzulegen und das Säurehalogenid zuzudosieren. Nach Beendigung der Reaktion filtriert man von ungelösten Anteilen, beispielsweise den Umsetzungsprodukten der gegebenenfalls eingesetzten Base mit dem Halogenwasserstoff, ab und engt das Filtrat ein. Aus der eingeengten Lösung kristallisiert sodann das gewünschte Reaktionsprodukt oder es kann durch Zusatz eines Alkohols, bevorzugt Methanol, ausgerührt und sodann abfiltriert werden. Für den Fall, dass als Lösungsmittel im erfindungsgemässen Verfahren von Anfang an ein Alkohol, beispielsweise Methanol, eingesetzt wurde, fällt das Reaktionsprodukt direkt als Kristallbrei an und kann durch einfache Filtration isoliert werden. Diese Verfahrensvariante ist wegen ihrer einfachen Durchführung bevorzugt.

Das Pivaloylessigsäure-2-chlor-5-[4-(2,4-di-tert.-amyl)-phenoxybutyramido]-anilid fällt im erfindungsgemässen Verfahren in hoher Reinheit an und kann ohne weitere Reinigung zur Herstellung von Farbfilmkupplern verwendet werden.

Es ist ausgesprochen überraschend, dass das gewünschte Reaktionsprodukt in Ausbeuten von über 85%, in vielen Fällen über 90% der theoretischen Ausbeute und in der beschriebenen hohen Reinheit anfällt, weil es sich bei den Ausgangsprodukten und in besonderem Masse beim Endprodukt um grosse Moleküle handelt, die eine Anzahl funktioneller Gruppen tragen, bei denen

unerwünschte Nebenreaktionen z.B. durch die entstehende Salzsäure zu befürchten waren. Hierzu sei beispielsweise auf den für niedermolekulare Stoffe hohen Wert von etwa 571 für das Molekulargewicht und auf die Anwesenheit von drei Carbonylgruppen, zweier aktiver N-H-Bindungen und die Anwesenheit zweier durch unterschiedliche Substituenten aktivierter aromatischer Kerne hingewiesen.

Beispiel 1

(Herstellung des Ausgangsproduktes)

In einer 1-Liter-Rührapparatur mit einer 30 cm langen verspiegelten Vigreux-Kolonne mit Kolonnenkopf werden 137 g Pivaloylessigsäuremethylester und 130 g Toluol zusammen mit 124,5 g 2-Chlor-5-nitro-anilin vorgelegt und unter Rühren zum Sieden erhitzt. Während 10 Stunden steigt die Sumpftemperatur von 120 bis 145°C an. Die Übergangstemperatur steigt von 64 bis ca. 110°C an. Es werden während der Destillationszeit etwa 112 ml Toluol-Methanol-Gemisch, gleichmässig ca. 10 bis 12 ml Destillat/St., abgenommen. Anschliessend wird der Kolbenrückstand mit Eiswasser unter Rühren abgekühlt, der entstandene dicke Kristallbrei abgesaugt und 3mal mit je ca. 30 ml kaltem Toluol gewaschen. Nach dem Trocknen im Vakuum bei 50°C verbleiben 190 g (90,9% der theoretischen Ausbeute, ber. auf Nitranilin) an Pivaloylessigsäure-2-chlor-5-nitro-anilid. Gelbe Kristalle Fp. 118–119°C.

Elementaranalyse:

| | C | H | N | Cl | O |
|---|---|---|---|---|---|
| Ber. | 52,3 | 5,0 | 9,4 | 11,9 | 21,5 |
| Gef. | 52,5 | 4,8 | 9,6 | 11,8 | 21,3 |
| | 52,4 | 4,7 | 9,6 | 11,7 | 21,6 |

Beispiel 2

a) 20 g Pivaloylessigsäure-2-chlor-5-nitro-anilid 100%ig werden mit 80 g Methanol, 2 g Pyridin und 2 g Ra-Nickel in einen 0,3-Liter-V4A-Autoklaven gegeben. Man gibt auf den Autoklaven 10 bar $H_2$ und hält die Temperatur durch Kühlen auf 25°C. Nach 1 ¼ h wird auf 40°C geheizt und bis zur Druckkonstanz hydriert. Man befreit vom Kontakt, wäscht mit wenig Methanol nach und gewinnt 108 g Lösung 16,7%ig an Pivaloylessigsäure-5-amino-2-chloranilid = 99,1% der theoretischen Ausbeute (Analyse durch Dünnschichtchromatographie und UV-Spektrum).

b) In ein 0,5-Liter-Rührgefäss werden 145 g Toluol und 37,0 g 2,4-Di-tert.-amyl-phenoxybuttersäurechlorid, 96,2%ig (0,105 Mol), gefüllt. Unter Rühren gibt man portionsweise 27,4 g festes Pivaloylessigsäure-5-amino-2-chloranilid (98%ig = 0,1 Mol), das nach a) erhalten wurde, zu. Die Lösung wird zum Sieden erhitzt, wobei Chlorwasserstoff entweicht. Nach ca. 15 Minuten beginnt man, Toluol abzudestillieren, wobei der Druck im Laufe der Destillation auf 20 mbar gesetzt wird. Zum heissen Rückstand gibt man 70 g Methanol und kühlt mit Eiswasser. Das ausgefallene Produkt wird abgesaugt und mehrfach mit Methanol gewaschen. Nach dem Trocknen gewinnt man 56 g Pivaloylessigsäure-2-chlor-5-[4-(2,4-di-tert.-amyl)-phenoxybutyramido]-anilid (97%ig) = 95% der theoretischen Ausbeute. (Gehalt wurde mittels potentiographischer Titration ermittelt.)

Beispiel 3

a) 10 g Pivaloylessigsäure-2-chlor-5-nitroanilid 100%ig werden mit 80 g Methanol, 2 g Pyridin und 1 g feuchtem Ra-Nickel (entspricht etwa 0,5 g trockenem Ra-Nickel) in einen 0,3 l V4A-Autoklaven gegeben. Man gibt auf den Autoklaven 10 bar $H_2$, wobei die Temperatur unter 25°C gehalten wird. Nach 1 ¼ h wird auf 40°C geheizt und bis zur Druckkonstanz hydriert. Man befreit die Lösung vom Kontakt, wäscht mit wenig Methanol und engt ein. Es verbleibt ein Rückstand von 9,1 g Pivaloylessigsäure-5-amino-2-chlor-anilid 96,4%ig = 97,5% der theoretischen Ausbeute. Der Reinheitsgrad wurde durch Dünnschicht-Chromatographie und UV-Spektroskopie bestimmt. Für die Weiterverarbeitung braucht die methanolische Lösung nicht eingeengt zu werden.

b) In ein 0,5-Liter-Rührgefäss werden 173,6 g methanolische Lösung des Pivaloylessigsäure-5-amino-2-chloranilids (0,1 Mol) nach a) mit 9,5 g Pyridin eingefüllt. Man kühlt auf 0–5°C und tropft innerhalb von 30–40 Minuten 37,0 g 4-(2,4-Di-tert.-amyl)-phenoxybuttersäurechlorid, 96,2%ig (0,105 Mol), ein. Man rührt 1,5 h bei 0°C nach und saugt den entstehenden Kristallbrei ab. Nach der Trocknung erhält man 54 g Pivaloylessigsäure-2-chlor-5-[4-(2,4-di-tert.-amyl)-phenoxybutyramido]-anilid (98%ig) = 93% der theoretischen Ausbeute (Gehaltsbestimmung analog Beispiel 2).

Beispiel 4

Die Umsetzung mit Wasserstoff kann auch anstatt der in den Beispielen 2a) und 3a) beschriebenen Varianten wie folgt ausgeführt werden:

10 g Pivaloylessigsäure-2-chlor-5-nitro-anilid 100%ig, 87 g Toluol, 2 g Pyridin und 0,4 g Ra-Nikkel werden in einen 0,3 l V4A-Autoklaven eingegeben. Dann wird auf 40°C geheizt und mit 30 bar $H_2$ bis zur Druckkonstanz hydriert. Man filtriert vom Kontakt und dampft das Lösungsmittel ab. Es verbleiben 9,1 g Pivaloylessigsäure-5-amino-2-chloranilid 97,2%ig = 98,3% der theoretischen Ausbeute.

Beispiel 5

Analog Beispiel 3a). Anstatt 2 g Pyridin wird 1 g Morpholin zugesetzt. Nach der Reduktion und nach Entfernen des Methanols verbleiben 18,4 g Pivaloylessigsäure-5-amino-2-chloranilid 97,6%ig = 99,8% der theoretischen Ausbeute.

Beispiel 6

200 g Pivaloylessigsäure-2-chlor-5-nitroanilid 100%ig werden mit 530 g Methanol, 4 g Pyridin und 20 g feuchtem Ra-Nickel (entspricht etwa 10 g trockenem Ra-Nickel) bei 25°C mit 25 bar $H_2$ bis zur Druckkonstanz hydriert. Es wird vom Kontakt filtriert und mit wenig Methanol gewaschen. Man gewinnt 769 g methanolische Lösung des Pi-

valoylessigsäure-5-amino-2-chloranilids mit einem Gehalt von 22,6% = 96,5% der theoretischen Ausbeute. Diese Lösung kann unmittelbar weiter nach Beispiel 3a) umgesetzt werden.

Beispiel 7 (zum Vergleich)

Reduktion von Pivaloylessigsäure-2-chlor-5-nitroanilid ohne Zusatz.

Die Reduktion wird ohne Pyridin analog Beispiel 2a) durchgeführt. Man gewinnt 18,2 g Produkt 85%ig = 86,0% der theoretischen Ausbeute. Das Produkt enthält ca. 8% 2,4-Dipivaloylacetamidochlorbenzol und ca. 3% 2,4-Diaminochlorbenzol. Der Reinheitsgrad und die Nebenprodukte wurden durch Dünnschicht-Chromatographie und UV-Spektroskopie bestimmt.

Beispiel 8

Die weitere Umsetzung der Aminoverbindung mit dem Säurechlorid kann auch anstatt der in den Beispielen 2b) und 3b) beschriebenen Varianten wie folgt ausgeführt werden:

27,4 g Pivaloylessigsäure-5-amino-2-chloranilid, 89%ig (0,1 Mol), werden mit 120 ml Essigsäureethylester und 11,2 g Triethylamin (0,11 Mol) auf -25°C gekühlt. Innerhalb von 30 Minuten werden 37,0 g 2,4-Di-tert.-amyl-phenoxybuttersäurechlorid eingetropft. Man erwärmt die Lösung bis auf Raumtemperatur und saugt dann ausgefallenes Triethylammoniumchlorid ab. Das Filtrat wird im Vakuum bei 90°C und auf 20 mbar reduziertem Druck eingeengt. In den heissen Rückstand werden 70 ml Methanol gegeben und dann kaltgerührt. Nach Absaugen und Trocknen gewinnt man 51 g Pivaloylessigsäure-2-chlor-5-[2,4-di-tert.-amyl-phenoxybutyramido]-anilid (96,7%ig) = 87,5% der theoretischen] Ausbeute. (Gehaltsbestimmung analog Beispiel 2.)

Beispiel 9

Analog Beispiel 8. Anstelle des Essigsäureethylesters werden 120 ml Toluol verwandt und anstelle des Triethylamins Dimethylbenzylamin. Ausbeute 51 g (98%ig) = 87,9% der theoretischen Ausbeute. (Gehaltsbestimmung analog Beispiel 2.)

**Patentansprüche**

1. Verfahren zur Herstellung von Pivaloylessigsäure-2-chlor-5-[4-(2,4-di-tert.-amyl)-phenoxybutyramido]-anilid, dadurch gekennzeichnet, dass man Pivaloylessigsäure-2-chlor-5-nitro-anilid mit Wasserstoff katalytisch in Gegenwart eines Amins oder Phosphins in einem inerten Lösungsmittel bei einer Temperatur von 0 bis 150°C umsetzt und die dabei erhältliche korrespondierende Aminoverbindung danach weiter mit einem Halogenid der 4-(2,4-Di-tert.-amyl)-phenoxybuttersäure bei einer Temperatur von -50 bis +150°C in einem organischen Lösungsmittel, gegebenenfalls in Gegenwart einer Base, umsetzt.

2. Verfahren nach Anspruch 1, dadurch gekennzeichnet, dass man die Umsetzung mit Wasserstoff bei einer Temperatur von 20 bis 80°C durchführt.

3. Verfahren nach Anspruch 1 und 2, dadurch gekennzeichnet, dass ein Amin eingesetzt wird.

4. Verfahren nach Anspruch 1 bis 3, dadurch gekennzeichnet, dass Pyridin eingesetzt wird.

5. Verfahren nach Anspruch 1, dadurch gekennzeichnet, dass die weitere Umsetzung mit einem Säurehalogenid bei einer Temperatur von -25 bis +110°C durchgeführt wird.

6. Verfahren nach Anspruch 1, dadurch gekennzeichnet, dass als Lösungsmittel für beide Verfahrensstufen ein Kohlenwasserstoff oder ein Alkohol eingesetzt wird.

7. Verfahren nach Anspruch 1 und 6, dadurch gekennzeichnet, dass man als Lösungsmittel Methanol benutzt.

8. Verfahren nach Anspruch 1, dadurch gekennzeichnet, dass man die weitere Umsetzung mit einem Säurehalogenid in Gegenwart einer organischen Base durchführt.

9. Verfahren nach Anspruch 1, dadurch gekennzeichnet, dass in einem Kohlenwasserstoff oder einem Alkohol als Lösungsmittel und in Gegenwart eines tertiären organischen Amins gearbeitet wird und nach der Umsetzung mit Wasserstoff ohne Zwischenisolierung der Aminoverbindung die weitere Umsetzung mit dem Säurehalogenid in der beschriebenen Lösung durchgeführt wird.

10. Verfahren nach Anspruch 1, dadurch gekennzeichnet, dass als Säurehalogenid das Säurechlorid eingesetzt wird.

**Claims**

1. Process for the preparation of pivaloylacetic acid 2-chloro-5-[4-(2,4-di-tert.-amyl)-phenoxybutyramido]-anilide, characterised in that pivaloylacetic acid 2-chloro-5-nitro-anilide is reacted catalytically with hydrogen, in the presence of an amine or phosphine, in an inert solvent, at a temperature of 0 to 150°C, and the corresponding amino compound obtainable by this reaction is then reacted further with a 4-(2,4-di-tert.-amyl)-phenoxybutyryl halide, at a temperature of -50 to +150°C, in an organic solvent, if appropriate in the presence of a base.

2. Process according to Claim 1, characterised in that the reaction with hydrogen is carried out at a temperature of 20 to 80°C.

3. Process according to Claim 1 and 2, characterised in that an amine is used.

4. Process according to Claim 1 to 3, characterised in that pyridine is used.

5. Process according to Claim 1, characterised in that the subsequent reaction with an acid halide is carried out at a temperature of -25 to +110°C.

6. Process according to Claim 1, characterised in that a hydrocarbon or an alcohol is used as the solvent for both process steps.

7. Process according to Claim 1 and 6, characterised in that methanol is used as the solvent.

8. Process according to Claim 1, characterised

in that the subsequent reaction with an acid halide is carried out in the presence of an organic base.

9. Process according to Claim 1, characterised in that the process is carried out in a hydrocarbon or an alcohol as the solvent and in the presence of a tertiary organic amine, and, after the reaction with hydrogen, without intermediate isolation of the amino compound, the subsequent reaction with the acid halide is carried out in the solution described.

10. Process according to Claim 1, characterised in that the acid chloride is used as the acid halide.

**Revendications**

1. Procédé pour la fabrication de 2-chloro-5-[4-(2,4-di-tert-amyl)-phénoxybutyramido]-anilide d'acide pivaloylacétique, caractérisé en ce que l'on fait réagir par voie catalytique le 2-choro-5-nitro-anilide d'acide pivaloylacétique avec l'hydrogène en présence d'une amine ou d'une phosphine dans un solvant inerte à une température de 0 à 150°C, et ensuite on fait encore réagir le composé amino correspondant ainsi obtenu avec un halogénure de l'acide 4-(2,4-di-tert-amyl)-phénoxybutyrique à une température de -50 à +150°C dans un solvant organique inerte, éventuellement en présence d'une base.

2. Procédé selon la revendication 1 caractérisé en ce que l'on effectue la réaction avec l'hydrogène à une température de 20 à 80°C.

3. Procédé selon la revendication 1 ou 2, caractérisé en ce que l'on utilise une amine.

4. Procédé selon la revendication 1 à 3, caractérisé en ce que l'on utilise la pyridine.

5. Procédé selon la revendication 1, caractérisé en ce que la réaction ultérieure avec un halogénure d'acide est effectuée à une température de -25 à +110°C.

6. Procédé selon la revendication 1, caractérisé en ce que l'on utilise comme solvant pour les deux étapes du procédé un hydrocarbure ou un alcool.

7. Procédé selon la revendication 1 à 6, caractérisé en ce que l'on utilise le méthanol comme solvant.

8. Procédé selon la revendication 1, caractérisé en ce que la réaction ultérieure avec un halogénure d'acide est effectuée en présence d'une base organique.

9. Procédé selon la revendication 1, caractérisé en ce que l'on opère dans un hydrocarbure ou un alcool comme solvant et en présence d'une amine organique tertiaire et, après la réaction avec l'hydrogène, la réaction ultérieure avec l'halogénure d'acide est effectuée dans la solution décrite sans isolement intermédiaire du composé amino.

10. Procédé selon la revendication 1 caractérisé en ce que l'on utilise comme halogénure d'acide le chlorure d'acide.